# EUROPEAN PATENT APPLICATION

(11) **EP 1 690 960 A2**
(43) Date of publication of application: **16.08.2006**
(21) Application number: 06002370.2
(22) Date of filing: 06.02.2006
(51) Int. Cl.: C23F 11/14, C23F 11/173, C07C 209/02, C07C 291/02

(54) **Corrosion inhibitors comprising nitrogen functionality**

(30) Priority: 15.02.2005 US 58007
(71) Applicant: Air Products and Chemicals, Inc., Allentown, PA 18195-1501 (US)
(72) Inventor: Goddard, Richard Joseph, Fogelsville, PA 18051 (US); Ford, Michael Edward, Trexlertown, PA 18087 (US)
(74) Representative: Kador & Partner

(57) **Abstract**

Compounds having N-oxide, quaternary ammonium, and/or betaine functionality according to formulas (1) - (4) are effective corrosion inhibitors, suitable for use in any of a variety of applications such as automotive cooling systems, heating boilers, drilling of oil wells, and the handling, transportation and storage of crude petroleum and various petroleum fractions.

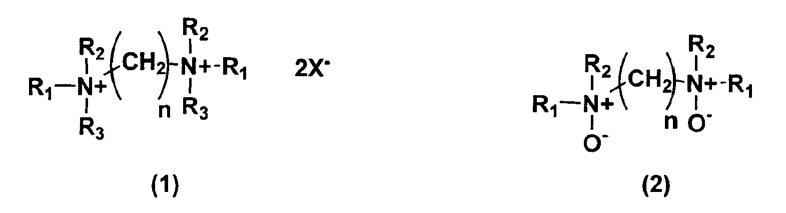

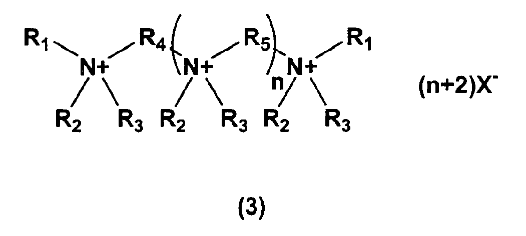

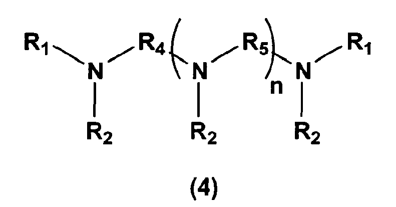

The groups R₁ - R₅ may be certain hydrocarbyl groups. In formulas (1) and (3), R₃ may also be (CH₂)ₚ-COO⁻ in which p is either 1 or 2, and R₂ may also be (CH₂)ₚ-COO⁻ in formulas (3) and (4). In the compounds of formulas (1) and (2), R₂ may also be a polyhydroxyalkyl group of formula (A), shown below.

## Description

### FIELD OF THE INVENTION

This invention relates to corrosion inhibitors. More particularly, it relates to corrosion inhibitors comprising nitrogen functionality.

### BACKGROUND OF THE INVENTION

Although significant effort has been directed toward development of corrosion-resistant alloys, prevention or inhibition of corrosion of metals remains a major concern. Reduction of corrosion is important in many applications such as automotive cooling systems, heating boilers, drilling of oil wells, and the handling, transportation and storage of crude petroleum and various petroleum fractions. In these and other applications, corrosion often can have a significant economic impact in terms of the maintenance, downtime, and replacement schedules required for the associated equipment. Thus, prevention or inhibition of corrosion minimizes unscheduled shutdowns, maintenance and repair, and provides for safer operation of the fluid handling equipment. To achieve this, one or more of additives referred to as corrosion inhibitors are typically incorporated into the corrosive process streams to reduce corrosion. Although many corrosion inhibitors are known in the art, satisfactory corrosion inhibition is not always obtainable with existing systems. Thus there is a continuing need for novel corrosion inhibitors suitable for use in some or all of the above-mentioned environments, or others.

### SUMMARY OF THE INVENTION

In one aspect, the invention provides a compound according to formula (1) 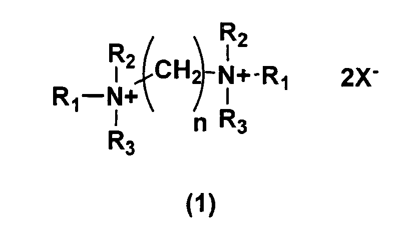 wherein R₁ is selected from the group consisting of C₄ - C₁₈ branched alkyl, linear alkyl, alkenyl, cycloalkyl, aryl, alkaryl, and aralkyl; R₂ is selected from the group consisting of C₁ - C₈ alkyl, alkenyl, cycloalkyl, aryl, alkaryl, aralkyl, and moieties of formula (A) 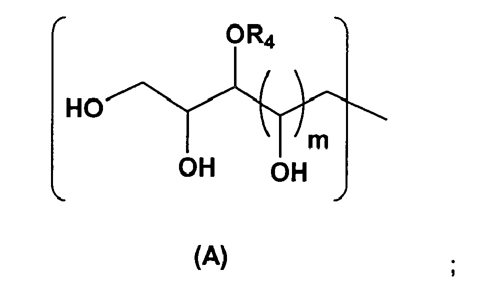 R₃ is C₁ - C₈ alkyl, alkenyl, cycloalkyl, aryl, alkaryl, or (CH₂)ₚ-COO⁻ in which p is either 1 or 2; n is an integer from 2 to 14; m is an integer from 0 to 2; R₄ is H, α-D-glucopyranosyl, β-D-pyranosyl, or β-D-galactopyranosyl; and X⁻ is Cl⁻, Br⁻, I⁻, OH⁻, CH₃COO⁻, 1/2 SO₄⁻² or 1/3 PO₄⁻³ provided that the number of X⁻ moieties in formula (1) is reduced by one for each R₃ that is (CH₂)ₚ-COO⁻; further provided that R₁ is not linear alkyl when R₃ is not (CH₂)ₚ-COO⁻.

In another aspect, the invention provides a compound according to formula (2) 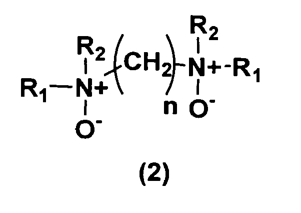 wherein R₁ is selected from the group consisting of C₄ - C₁₈ branched alkyl, linear alkyl, alkenyl, cycloalkyl, aryl, alkaryl, and aralkyl; R₂ is selected from the group consisting of C1 - C8 alkyl, alkenyl, cycloalkyl, aryl, alkaryl, aralkyl, and moieties of formula (A) 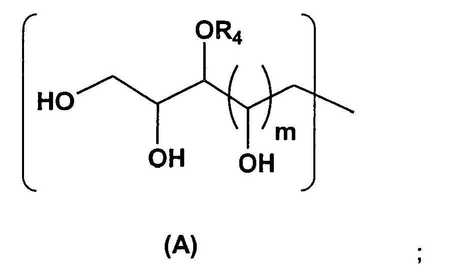 n is an integer from 2 to 14; m is an integer from 0 to 2; and R₄ is H, α-D-glucopyranosyl, β-D-pyranosyl, or β-D-galactopyranosyl.

In yet another aspect, the invention provides a compound according to formula (3) 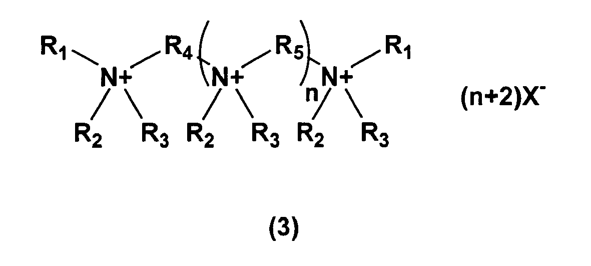 wherein R₁ is C₃ - C₁₂ branched alkyl, linear alkyl, alkenyl, cycloalkyl, or aryl-substituted branched or linear alkyl; R₂ and R₃ are each individually selected from the group consisting of C₁ - C₄ alkyl, benzyl, C₃ - C₅ alkenyl, and (CH₂)ₚ-COO⁻ in which p is either 1 or 2, provided that not both of R₂ and R₃ are (CH₂)ₚ-COO⁻; R₄ and R₅ are chosen independently from C₂ - C₆ alkylene; n is an integer from 0 to 4; and X⁻ is Cl⁻, Br⁻, I⁻, OH⁻, CH₃COO⁻, 1/2 SO₄⁻², or 1/3 PO₄⁻³, provided that the number of X⁻ moieties in formula (3) is reduced by one for each R₃ that is (CH₂)ₚ-COO⁻; further provided that, if n is 0 and R₄ is (CH₂)₂, then either R₁ is not linear alkyl or R₃ is (CH₂)ₚ-COO⁻, or both.

In a further aspect, the invention provides a compound according to formula (4) 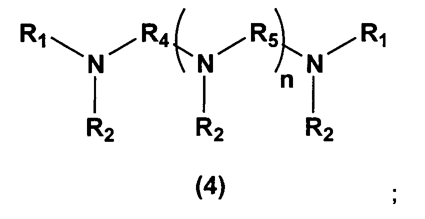 wherein R₁ is C₃ - C₁₂ branched alkyl, linear alkyl, alkenyl, cycloalkyl, or aralkyl; R₂ is selected from the group consisting of C₁ - C₄ alkyl, benzyl, C₃ - C₅ alkenyl, and (CH₂)ₚ-COO⁻ in which p is either 1 or 2; R₄ and R₅ are chosen independently from C₂ - C₆ alkylene; and n is an integer from 0 to 4; provided that n is ≥ 1 if R₂ is not (CH₂)ₚ-COO⁻.

In a still further aspect, the invention provides a method of reducing corrosion of a metal surface, comprising contacting the metal surface with a corrosive medium comprising a corrosion inhibitor according to any of formulas (1), (2), (3), and (4) shown above.

### DETAILED DESCRIPTION OF THE INVENTION

This invention provides certain substituted alkylenediamines or polyamines, and derivatives of these, suitable for use in preventing or inhibiting corrosion of metal surfaces in aqueous or other corrosive environments. Four classes of compounds are provided, identified below as formulas (1) - (4). Each of these will now be discussed in detail.

The first class of new corrosion inhibitors consists of certain derivatives of N,N'-dialkylalkylenediamines as shown below in formula (1).

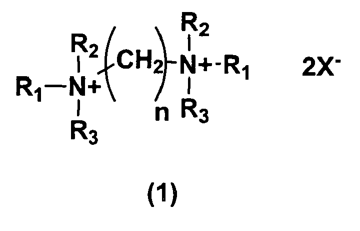

In formula (1), R₁ is selected from the group consisting of C₄ - C₁₈ branched alkyl, linear alkyl, alkenyl, cycloalkyl, aryl, alkaryl, and aralkyl; R₂ is selected from the group consisting of C₁ - C₈ alkyl, alkenyl, cycloalkyl, aryl, alkaryl, aralkyl and moieties of formula (A) 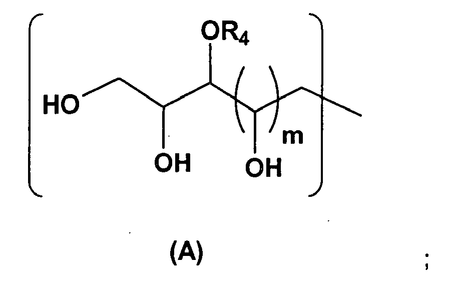 R₃ is C₁ - C₈ alkyl, alkenyl, cycloalkyl, aryl, alkaryl, or (CH₂)ₚ-COO⁻ in which p is either 1 or 2; n is an integer from 2 to 14; m is an integer from 0 to 2; R₄ is H, α-D-glucopyranosyl, β-D-pyranosyl, or β-D-galactopyranosyl; and X⁻ is Cl⁻, Br⁻, I⁻, OH⁻, CH₃COO⁻, 1/2 SO₄⁻², or 1/3 PO₄⁻³, provided that the number of X⁻ moieties in formula (1) is reduced by one for each R₃ that is (CH₂)ₚ-COO⁻; further provided that R₁ is not linear alkyl when R₃ is not (CH₂)ₚ-COO⁻. Substituents of formula (A) above, regardless of the identity of R₄, will be referred to herein as "polyhydroxyalkyl" groups. It will be understood to the artisan of ordinary skill in the art that the use of the term "2X⁻" in formula (1) and in formula 1(a) below is intended to indicate the amount of anion needed to provide charge neutrality.

Suitable exemplary R₁ groups include straight-chain or branched-chain alkyl groups such as n-butyl, 2-butyl, *tert-*butyl*,* isobutyl, n-pentyl, 2-pentyl, *tert*-pentyl, isopentyl, neopentyl, 2-methylpentyl, n-hexyl, isohexyl, heptyl, 2-ethylhexyl, octyl, nonyl, 3,5-dimethyloctyl, 3,7-dimethyloctyl, decyl, undecyl, dodecyl, tridecyl, tetradecyl, 3-methyl-10-ethyldodecyl, pentadecyl, hexadecyl, heptadecyl, octadecyl, cocoalkyl (C₈H₁₇ - C₁₆H₃₃), and tallow (C₁₆H₃₃ - C₁₈H₃₇), as well as aralkyl, aryl, alkaryl, alicyclic, bicyclic, and similar groups. Examples of such groups are cyclohexylmethyl, benzyl, pinyl, pinylmethyl, phenethyl, *p*-methylbenzyl, phenyl, tolyl, xylyl, naphthyl, ethylphenyl, methylnaphthyl, dimethylnaphthyl, norbornyl, and norbornylmethyl.

Suitable exemplary R₂ and R₃ groups include methyl, ethyl, n-propyl, isopropyl, n-butyl, 2-butyl, *tert*-butyl, isobutyl, n-pentyl, 2-pentyl, *tert*-pentyl, isopentyl, neopentyl, 2-methylpentyl, n-hexyl, isohexyl, heptyl, 2-ethylhexyl, octyl, cyclohexylmethyl, benzyl, phenethyl, *p*-methylbenzyl, phenyl, tolyl, xylyl, and ethylphenyl. Suitable exemplary R₂ groups also include polyhydroxyalkyl groups such as 1-deoxyglucityl, 2,3-dihydroxypropyl, and analogous 1-deoxy groups derived from mannitol, xylitol, galactitol, maltitol, and lactitol.

Compounds according to formula (1) may be prepared by any method known in the synthetic organic chemical art. For example, they may be prepared by reaction of the corresponding N,N'-di(R₁)-N,N'-di(R₂)alkylenediamine with an alkyl halide to introduce the group R₃, according to methods well known in the chemical art. In the case where R₃ is (CH₂)ₚ-COO-, reaction with a halocarboxylic acid salt may be used, for example a salt of a haloacetic acid (see, e.g., Example 1 of U.S. Pat. No. 3,839,425; to E. I. DuPont de Nemours and Company).

If R₂ is a polyhydroxyalkyl group, a number of variations of R₂ are possible, depending upon the value of R₄ and m, to form a variety of compounds according to formula (1a) below.

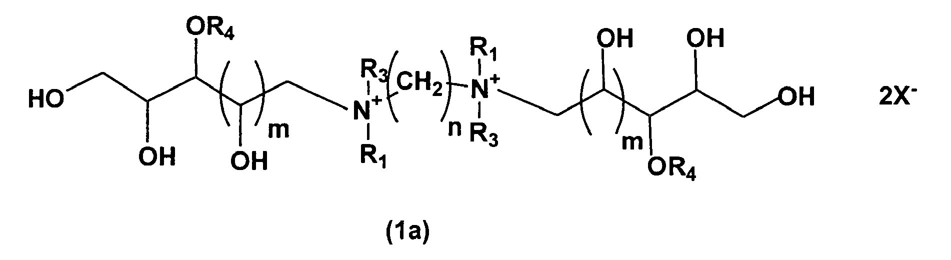

Compounds according to formula (1a) may be obtained by any suitable procedure, for example that reported by V. I. Veksler, et. al., Zhurnal Obshchei Khimii, 50(9), 2120-2123 (1980). Although any of a variety of polyhydroxyalkyl groups may be incorporated in compounds according to the invention, they most typically will be derived from the open-chain forms of reducing sugars, for example glucose. Exemplary polyhydroxyalkyl groups are derived from glucose; i.e., they are 1-deoxyglucityl groups. In general, polyhydroxyalkyl groups of N-(polyhydroxyalkyl)alkylamines useful for making N,N'-dialkyl-N,N'-bis(polyhydroxyalkyl)alkylenediamines according to the invention may be derived from any of the group of reducing sugars consisting of glucose, fructose, maltose, lactose, galactose, mannose, and xylose. Typically, the reducing sugar will be an aldose, although ketoses may also be used, and both monosaccharides and disaccharides may be used, with convenient sources of the latter including high dextrose corn syrup, high fructose corn syrup, and high maltose corn syrup. Other useful polyhydroxyalkyl groups may be derived from glyceraldehydes. In some embodiments, R₂ is a polyhydroxyalkyl group derived from glucose; i.e. the group is 1-deoxyglucityl. In this case, m is 2 and R₄ is hydrogen.

The second class of corrosion inhibitors includes bis N-oxides of general formula (2) 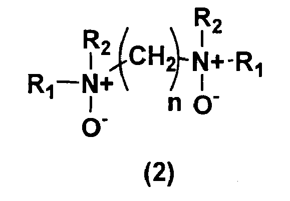 where R₁ and R₂ are as defined above in relation to compounds of formula (1).

Compounds according to formula (2) may be prepared by any method known in the synthetic organic chemical art. For example, they may be prepared by treatment of the corresponding ditertiary amine with an oxidizing agent such as hydrogen peroxide (see, e.g., U.S. Pat. No. 5,710,332, Example 4, for preparation of N-oxides of N,N-dialkylglucamines).

The case where R₂ is a polyhydroxyalkyl group constitutes a subclass of formula (2), shown below as formula (2a), wherein R₁, R₄, m, and n have the same meanings as defined above in relation to formula (1a). 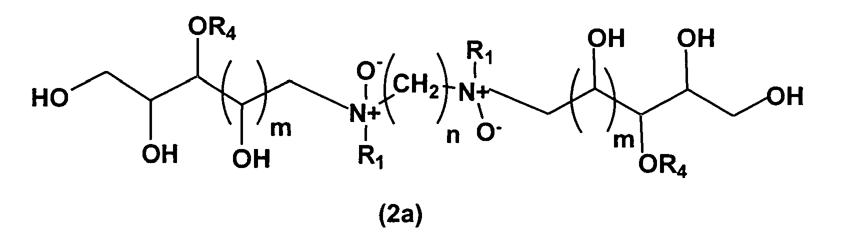 In one embodiment, the polyhydroxyalkyl group is derived from glucose; i.e. the group is 1-deoxyglucityl. In this case, m is 2 and R₄ is hydrogen.

The third class of corrosion inhibitors consists of quaternary derivatives of N,N'-dialkyl polyalkylene polyamines of general formula (3).

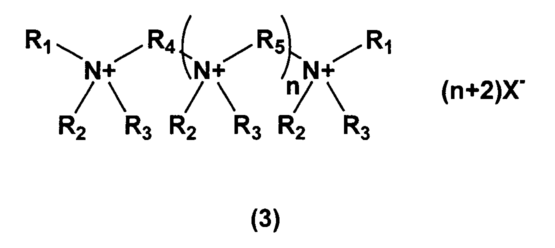

In formula (3), R₁ is C₃ - C₁₂ branched alkyl, linear alkyl, alkenyl, cycloalkyl, or aryl-substituted branched or linear alkyl; R₂ and R₃ are each individually selected from the group consisting of C₁ - C₄ alkyl, benzyl, C₃ - C₅ alkenyl, and (CH₂)_{P}-COO⁻ in which p is either 1 or 2, provided that not both of R₂ and R₃ are (CH₂)_{P}-COO⁻; R₄ and R₅ are chosen independently from C₂-C₆ alkylene; n is an integer from 0 to 4; and X⁻ is Cl⁻, Br⁻, I⁻, OH⁻, CH₃COO⁻, 1/2 SO₄⁻², or 1/3 PO₄⁻³, provided that the number of X⁻ moieties in formula (3) is reduced by one for each R₃ that is (CH₂)ₚ-COO⁻. It will be understood to the artisan of ordinary skill in the art that the use of the term "(n+2)X⁻" in formula (3) is intended to indicate the amount of anion needed to provide charge neutrality.

In some embodiments of the invention, if n is 0 and R₄ is (CH₂)₂, then either R₁ is not linear alkyl or R₃ is (CH₂)ₚ-COO⁻, or both. Suitable exemplary R₁ groups include straight-chain or branched-chain alkyl groups such as n-propyl, isopropyl, n-butyl, 2-butyl, *tert*-butyl, isobutyl, n-pentyl, 2-pentyl, *tert*-pentyl, isopentyl, neopentyl, 2-methylpentyl, n-hexyl, isohexyl, heptyl, 2-ethylhexyl, octyl, nonyl, 3,5-dimethyloctyl, 3,7-dimethyloctyl, decyl, undecyl, and dodecyl, as well as cyclohexylmethyl, benzyl, phenethyl, and *p*-methylbenzyl. Suitable exemplary R₂ and R₃ groups include methyl, ethyl, n-propyl, isopropyl, n-butyl, 2-butyl, *tert*-butyl, isobutyl, and benzyl. In some embodiments, R₄ and R₅ are both (CH₂)₂, (CH₂)₃, (CH₂)₆, or (CH₂)₃CH(CH₃)CH₂.

Compounds according to formula (3) may be prepared by any method known in the synthetic organic chemical art. For example, they may be prepared by alkylation under conditions known in the art of the corresponding polyamine (4), which may in turn be obtained for example according to procedures of any of U.S. Pat. Nos. 4,126,640; 4,195,152; or 6,015,852; 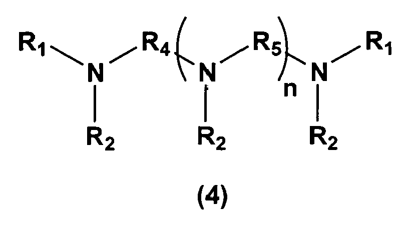 wherein R₁ - R₅ are as defined for formula (3). Such alkylation may for example be performed via reaction with an alkyl halide (see, e.g., Synthetic Organic Chemistry, R. B. Wagner and H. D. Zook, John Wiley and Sons, New York, 1953, p. 668). Compounds according to formula (4) themselves constitute a fourth class of corrosion inhibitors according to the invention, with R₁ - R₅ and n being as defined above in relation to formula (3). In some embodiments, n is ≥ 1 if R₂ is not (CH₂)ₚ-COO⁻- In some embodiments, R₄ and R₅ are both (CH₂)₂, (CH₂)₃, (CH₂)₆, or (CH₂)₃CH(CH₃)CH₂.

The inhibitors of this invention can provide protection against corrosion to metals either on their own, or in any desired mixture with one another or with other inhibitors or inert or cooperative materials. Nonlimiting examples of such other ingredients include neutralizing amines, fatty amines, quaternary ammonium salts, imidazolines, imidazoles, alkynols, phosphonic acids, defoamers, and water or other solvents. The amount of the inhibitors of this invention in such a mixture can be varied over a range of 1 to 100 wt%, preferably 5 to 50 wt%. Typically, mixtures containing the inhibitors are added at a concentration of 1 to 5000 ppm by weight relative to a corrosive medium that contacts the metal surface, although concentrations in the range from 1 to 100 ppm are more typical. Corrosive media may be aqueous or liquid organic media, or they may contain both aqueous and organic components in a mixture that may be a solution, a suspension, or a combination of these. Examples of corrosive media that may be treated with the corrosion inhibitors include automotive cooling systems, heating boilers, drilling of oil wells, and the handling, transportation, and storage of crude petroleum and various petroleum fractions.

A typical aqueous corrosion inhibitor formulation for treatment of boiler water or steam condensates that includes the corrosion inhibitors of the invention may include the following components by weight:
1-20% compounds according to any of formulas (1), (2,), (3), and (4);
4-40% of one or more basic neutralizing compounds, for example NaOH, KOH, phosphates, silicates, carbonates, borates, ammonia, ammonium salts, neutralizing amines, and volatile amines such as cyclohexylamine, morpholine, dicyclohexylamine, and diethylaminoethanol;
0-20% film-forming amines such as octadecylamine, tallowamines, cocoamines, oleylamines, amidoamines, imidazolines, fatty alkylamines or diamines, and quaternary salts of these or salts of these with organic or inorganic acids;
0-10% one or more other active ingredients such as oxygen scavengers, scale inhibitors, chelating agents, dispersants, emulsifiers, surfactants, and water-soluble polymers; and
10-95% water.

A typical corrosion inhibitor formulation for metal working, metal treatment or metal lubricants that includes the corrosion inhibitors of the invention may include the following components by weight:
30-99% one or more carriers or solvents, for example hydrocarbon solvents or oils or waxes, mineral oils or waxes, synthetic esters or polyesters, polyethers, alcohols, water, and natural fats or oils such as tall oil, rapeseed oil, corn oil, castor oil, cottonseed oil, palm oil, lard oil, and tallow;
1-20% compounds according to any of formulas (1), (2,), (3), and (4);
0-20% co-corrosion Inhibitors such as organic acids and their mineral or amine salts, organo-boron compounds or salts, octadecylamine, tallowamines, cocoamines, oleylamines, amidoamines, imidazolines, alkanolamides, sulfonamides, fatty alkylamines or diamines, and quaternary salts of these or salts of these with organic or inorganic acids;
0-20% basic neutralizing compounds such as neutralizing amines, alkylamines, alkylalkanolamines, alkanolamines, and alkaline salts; and
0-10% one or more other active ingredients such as water repellents, antioxidants, lubricants, emulsifiers, surfactants, defoamers, biocides, and stabilizers.

A typical corrosion inhibitor formulation for oil field, petroleum transportation, or refinery applications that includes the corrosion inhibitors of the invention may include the following components by weight:
20-99% one or more carriers or solvents such as hydrocarbon solvents or oils, water, and dispersing or coupling agents such as glycols, glycol ethers, alcohols, esters, ketones, amides, surfactants, emulsifiers, or dispersants;
1-30% compounds according to any of formulas (1), (2,), (3), and (4);
0-30% one or more co-corrosion inhibitors, such as organic acids and their mineral or amine salts, organophosphonates, phosphate esters, alkynols; neutralizing inhibitors such as alkaline salts, ammonia, ammonium salts, and neutralizing amines; volatile amines such as cyclohexylamine, morpholine, alkylamines, alkylalkanolamines and alkanolamines; octadecylamine, tallowamines, cocoamines, oleylamines, amidoamines, imidazolines, alkyl pyridines, amides and their salts, fatty alkylamines or diamines, and quaternary salts of these or salts of these with organic or inorganic acids; and
0-20% one or more other active ingredients such as nonemulsifiers, antifoams, surfactants, biocides, oxygen scavengers, hydrogen sulfide scavengers, and scale inhibitors.

In addition to their activity as corrosion inhibitors, compounds (1a) and (2a) of the present invention may exhibit increased biodegradation and lower aquatic toxicity relative to inhibitors of the prior art. More generally, inhibitors (1), (2), (3), and (4) of the present invention, being diamine or polyamine compounds, may exhibit lower aquatic toxicity relative to some existing inhibitors.

The invention is further illustrated by the following examples, which are presented for purposes of demonstrating, but not limiting, the methods and compositions of this invention.

### EXAMPLES

Example 1 illustrates preparation of N,N'-dimethyl-N,N'-dilaurylethylenediamine, an example of an intermediate in the synthesis of class corrosion inhibitors of formula (1). Examples 2-10 illustrate preparation of other N,N'-di(R₁)-N,N'-di(R₂)alkylenediamines.

### Examples 1-10

A 300 mL Autoclave Engineers stainless steel reactor was charged with 72.4 g (0.40 mole) lauronitrile, 16.8 g (0.19 mole) of N,N'-dimethylethylenediamine, 1.45 g (dry weight basis) of a 5% palladium-on-carbon catalyst, and 48 g of isopropanol. The reactor was closed, purged with nitrogen and hydrogen, and pressurized to about 600 psig with hydrogen. The mixture was heated with stirring (1000 rpm) to 125°C, pressurized with hydrogen to 1000 psig, and maintained at this temperature and pressure via regulated hydrogen feed. After 7 hr, the mixture was cooled to room temperature and the product was removed from the reactor with filtering through an internal 0.5µm sintered metal element. Analysis of the product by GC (Gas Chromatography) and GC-MS (Gas Chromatography-Mass Spectrometry) indicated that conversion was complete, and that the product consisted of 98+% N,N'-dimethyl-N,N'-dilaurylethylenediamine and just over 1% of N,N'-dimethyl-N-laurylethylenediamine. Vacuum distillation (190 - 200°C/80 - 100 Torr) provided pure N,N'-dimethyl-N,N'-dilaurylethylenediamine. Additional N,N'-di(R₁)-N,N'-di(R₂)alkylenediamines may be prepared and characterized using procedures similar to those described above. Some of these diamines are shown as Examples 2-10 in Table 1.

**TABLE 1**

| | | | |
|---|---|---|---|
| 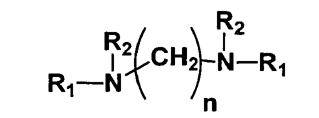 | | | |

| Example | R₁ | R₂ | n |
|---|---|---|---|
| 1 | C₁₂H₂₅ | CH₃ | 2 |
| 2 | C₆H₁₁ | C₂H₅ | 2 |
| 3 | C₆H₁₁ | C₂H₅ | 6 |
| 4 | C₈H₁₅ | CH₃ | 2 |
| 5 | C₈H₁₅ | CH₃ | 6 |
| 6 | C₁₀H₂₁ | CH₃ | 2 |
| 7 | C₁₂H₂₅ | C₂H₅ | 2 |
| 8 | Cocoalkyl | CH₃ | 2 |
| | (C₈H₁₇ - C₁₆H₃₃) | | |
| 9 | Cocoalkyl | C₂H₅ | 2 |
| | (C₈H₁₇ - C₁₆H₃₃) | | |
| 10 | Tallow | CH₃ | 2 |
| | (C₁₆H₃₃ - C₁₈H₃₇) | | |

Examples 11 - 15 illustrate preparation of bis betaine salts, i.e., examples of the class (1) of corrosion inhibitor where R₃ is (CH₂)ₚ-COO⁻, and specifically of Example 11 in Table 2.

### Examples 11 - 15

To a 250 mL three-necked flask equipped with a magnetic stirrer, reflux condenser, thermometer and nitrogen purge were added 20 g (0.0472 mole) of N,N'-dimethyl-N,N'-dilaurylethylenediamine, 25.54 g (0.1180 mole) of sodium iodoacetate, 80 mL of isopropanol, and 8 mL of deionized water at ambient temperature. The mixture was heated with stirring to 80°C and maintained at that temperature for 4.5 hrs. After cooling to room temperature, the solvent was removed under vacuum with a rotary evaporator. Addition of isopropanol (about 100 mL), vacuum filtration, and subsequent removal of isopropanol under vacuum with a rotary evaporator yielded pure N,N'-di(carboxymethyl)-N,N'-dimethyl-N,N'-dilaurylethylenediammonium dihydroxide inner salt. Additional bis betaines may be prepared and characterized using procedures similar to those described above. Some of these are shown in Table 2, wherein X is I in all examples.

**TABLE 2**

| | | | | |
|---|---|---|---|---|
| 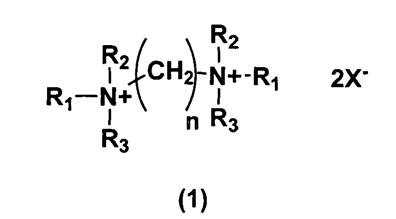 | | | | |

| Example | R₁ | R₂ | R₃ | n |
|---|---|---|---|---|
| 11 | C₁₂H₂₅ | CH₃ | CH₂COO⁻ | 2 |
| 12 | C₆H₁₁ | C₂H₅ | CH₂COO⁻ | 2 |
| 13 | C₈H₁₅ | CH₃ | CH₂COO⁻ | 2 |
| 14 | Cocoalkyl | CH₃ | CH₂COO⁻ | 2 |
| | (C₈H_{I7} - C₁₆H₃₃) | | | |
| 15 | Tallow | CH₃ | CH₂COO⁻ | 2 |
| | (C₁₆H₃₃ - C₁₈H₃₇) | | | |

Examples 16 -20 illustrate preparation of several bis N-oxides, examples of class (2) corrosion inhibitors in which none of the substituents on either nitrogen is a polyhydroxyalkyl group.

### Examples 16 - 20

To a 100 mL three-necked flask equipped with a magnetic stirrer, reflux condenser, thermometer, septum and nitrogen purge were added 5 g (0.0118 mole) of N,N'-dimethyl-N,N'-laurylethylenediamine and 25 mL of isopropanol at ambient temperature. The mixture was heated with stirring to 60°C, after which 3.2 g (0.028 mole; 2.4 eq.) of 30 wt% aqueous hydrogen peroxide was added dropwise from a syringe. The reaction was maintained at 60°C for 6 hrs. After cooling to room temperature, removal of the solvent under vacuum with a rotary evaporator yielded N,N'-dimethyl-N,N'-dilauryiethylenediamine-N,N'-bis(N-oxide). The identity and purity of the product were determined by NMR analysis. Additional analogous alkylenediamine bis(N-oxides) may be prepared and characterized using procedures similar to those described above. Some of these are shown in Table 3.

**TABLE 3**

| | | | |
|---|---|---|---|
| 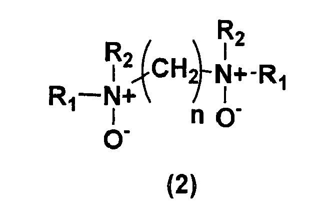 | | | |

| Example | R₁ | R₂ | n |
|---|---|---|---|
| 16 | C₁₂H₂₅ | CH₃ | 2 |
| 17 | C₆H₁₁ | C₂H₅ | 2 |
| 18 | C₈H₁₅ | CH₃ | 2 |
| 19 | Cocoalkyl | CH₃ | 2 |
| | (C₈H₁₇ - C₁₆H₃₃) | | |
| 20 | Tallow | CH₃ | 2 |
| | (C₁₆H₃₃ - C₁₈H₃₇) | | |

Examples 21 - 24 illustrate preparation of several bis N-oxides in which both nitrogen atoms are substituted with polyhydroxyalkyl groups; i.e. compounds of subclass (2a).

### Examples 21 - 24

The procedure of Example 20 is repeated with N,N'-dioctyl-N,N'-bis(1-deoxyglucityl)ethylenediamine, with use of methanol rather than isopropanol as solvent, to prepare the compounds shown in Table 4, where R₄ = H and m and n are both 2.

**TABLE 4**

| | |
|---|---|
| 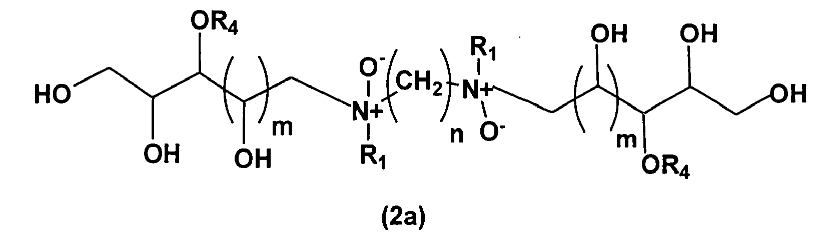 | |

| Example | R₁ |
|---|---|
| 21 | C₈H₁₇ |
| 22 | C₆H₁₁ |
| 23 | C₄H₉ |
| 24 | C₁₂H₂₅ |

Examples 25 and 26 illustrate the use of certain exemplary compounds of the invention as corrosion inhibitors.

### Example 25 - Control (no inhibitor)

Rates of corrosion were determined by an electrochemical potentiodynamic technique. The experimental apparatus was a standard three-electrode electrochemical cell with the specimen or working electrode fabricated from 1018 carbon steel solid rod. A solution to working electrode surface area ratio of 190 mL/cm² was used for testing. Polarization was started at 250 mV below (more active than) open circuit potential (E_{oc} after 1 hr) and scanned in the positive direction at a rate of 0.6 V/hour to 250 mV above E_{oc}. Current was recorded continuously. Corrosion potential (E_{corr}) and corrosion current (I_{corr}) were derived from the polarization curve (E vs. Log I) using Tafel Analysis. The corrosion rate, expressed in mils per year (mpy), was calculated from the corrosion current. Corrosion testing was performed using a simulated brine solution, prepared by mixing 85.07 g calcium chloride dihydrate, 39.16g magnesium chloride hexahydrate, 2025.00g sodium chloride, and 19L of distilled water according to NACE Test Method 1 D196. For this example, the volume of simulated brine solution required for a solution to working electrode surface area ratio of 190 mL/cm² was held in the electrochemical testing apparatus at a temperature of 120 (± 2) °F. The brine solution was sparged continuously with gaseous carbon dioxide for 2 hours prior to and during testing. With no added inhibitor, a corrosion rate of 47.7 mpy was determined for a test substrate of 1018 carbon steel.

### Example 26 - Corrosion Inhibitors of Classes (1), (2), (3), and (4)

Corrosion performance is determined using the general procedure of Example 25, but 100 ppm of a compound of Type (1), (1a), (2), (2a), (3) or (4), by weight relative to the brine solution is added to the electrochemical testing apparatus prior to measurement. A corrosion rate of <25 mpy is determined for a test substrate of 1018 carbon steel.

### Uses of Compounds of Formula (1) - (4)

Compounds according to any of the four classes described above may be incorporated into compositions including any of a wide variety of other ingredients designed to complement their utility as corrosion inhibitors. The performance properties of such products may be optimized for a specific application by appropriate modification of the structure of the inhibitor and the choice of the substituents R₁, R₂, R₃, R₄, and R₅, the number of repeating units m or n in the linking groups, and the length of the alkylene groups between the nitrogen atoms. Such optimization is routine, and within the ability of the person of ordinary skill in the art in the particular application area. Thus manipulation of these variables yields compounds which may find utility as corrosion inhibitors in a variety of applications, including for example those outlined in the foregoing disclosure.

Although the invention is illustrated and described herein with reference to specific embodiments, it is not intended that the subjoined claims be limited to the details shown. Rather, it is expected that various modifications may be made in these details by those skilled in the art, which modifications may still be within the spirit and scope of the claimed subject matter and it is intended that these claims be construed accordingly.

## Claims

1. A compound according to formula (1) 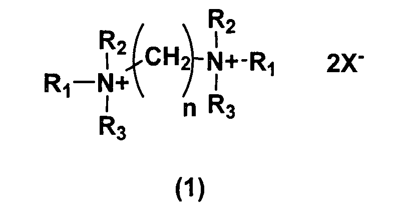 wherein R₁ is selected from the group consisting of C₄ - C₁₈ branched alkyl, linear alkyl, alkenyl, cycloalkyl, aryl, alkaryl, and aralkyl; R₂ is selected from the group consisting of C₁ - C₈ alkyl, alkenyl, cycloalkyl, aryl, alkaryl, aralkyl, and moieties of formula (A) 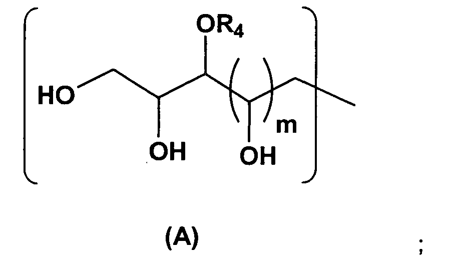 R₃ is C₁ - C₈ alkyl, alkenyl, cycloalkyl, aryl, alkaryl, or (CH₂)ₚ-COO⁻ in which p is either 1 or 2; n is an integer from 2 to 14; m is an integer from 0 to 2; R₄ is H, α-D-glucopyranosyl, β-D-pyranosyl, or β-D-galactopyranosyl; and X⁻ is Cl⁻, Br⁻, I⁻, OH⁻, CH₃COO⁻, 1/2 SO₄⁻², or 1/3 PO₄⁻³, provided that the number of X⁻ moieties in formula (1) is reduced by one for each R₃ that is (CH₂)ₚ-COO⁻; further provided that R₁ is not linear alkyl when R₃ is not (CH₂)ₚ-COO⁻.

2. The compound of claim 1, wherein R₁ is a moiety of formula (A).

3. The compound of claim 1, wherein R₁ is dodecyl.

4. The compound of claim 1, wherein R₁ is cocoalkyl.

5. The compound of claim 1, wherein R₁ is tallow.

6. The compound of claim 1, wherein R₂ is 1-deoxyglucityl.

7. The compound of claim 1, wherein R₂ is methyl or ethyl.

8. The compound of claim 1, wherein R₃ is (CH₂)p-COO⁻.

9. The compound of claim 1, wherein n is 2 or 6.

10. A compound according to formula (2) 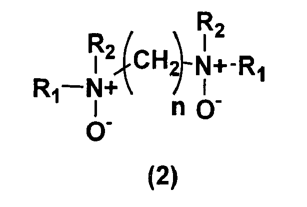 wherein R₁ is selected from the group consisting of C₄ - C₁₈ branched alkyl, linear alkyl, alkenyl, cycloalkyl, aryl, alkaryl, and aralkyl; R₂ is selected from the group consisting of C1 - C8 alkyl, alkenyl, cycloalkyl, aryl, alkaryl, aralkyl, and moieties of formula (A) 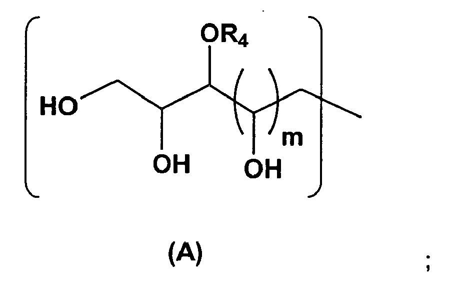 n is an integer from 2 to 14; m is an integer from 0 to 2; and R₄ is H, α-D-glucopyranosyl, β-D-pyranosyl, or β-D-galactopyranosyl.

11. The compound of claim 10, wherein R₁ is dodecyl.

12. The compound of claim 10, wherein R₁ is cocoalkyl.

13. The compound of claim 10, wherein R₁ is tallow.

14. The compound of claim 10, wherein R₂ is methyl or ethyl.

15. The compound of claim 10, wherein R₂ is a moiety of formula (A).

16. The compound of claim 10, wherein R₂ is 1-deoxyglucityl.

17. The compound of claim 16, wherein R₁ is selected from the group consisting of butyl, hexyl, octyl, and dodecyl.

18. The compound of claim 10, wherein n is 2 or 6.

19. A compound according to formula (3) 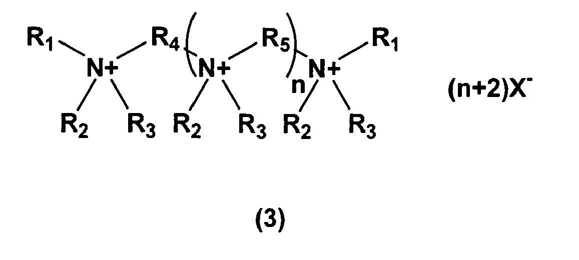 wherein R₁ is C₃ - C₁₂ branched alkyl, linear alkyl, alkenyl, cycloalkyl, or aryl-substituted branched or linear alkyl; R₂ and R₃ are each individually selected from the group consisting of C₁ - C₄ alkyl, benzyl, C₃ - C₅ alkenyl, and (CH₂)ₚ-COO⁻ in which p is either 1 or 2, provided that not both of R₂ and R₃ are (CH₂)ₚ-COO⁻; R₄ and R₅ are chosen independently from C₂ - C₆ alkylene; n is an integer from 0 to 4; and X⁻ is Cl⁻, Bi, I⁻, OH⁻, CH₃COO-, 1/2 SO₄⁻², or 1/3 PO₄⁻³, provided that the number of X⁻ moieties in formula (3) is reduced by one for each R₃ that is (CH₂)ₚ-COO⁻; further provided that, if n is 0 and R₄ is (CH₂)₂, then either R₁ is not linear alkyl or R₃ is (CH₂)ₚ-COO⁻, or both.

20. The compound of claim 19, wherein R₁ is branched C₃ - C₁₂ alkyl.

21. The compound of claim 19, wherein R₃ is (CH₂)_{P}-COO⁻.

22. The compound of claim 19, wherein R₄ and R₅ are both (CH₂)₂.

23. The compound of claim 19, wherein R₄ and R₅ are both (CH₂)₃.

24. The compound of claim 19, wherein R₄ and R₅ are both (CH₂)₆.

25. The compound of claim 19, wherein R₄ and R₅ are both (CH₂)₃CH(CH₃)CH₂.

26. A compound according to formula (4) 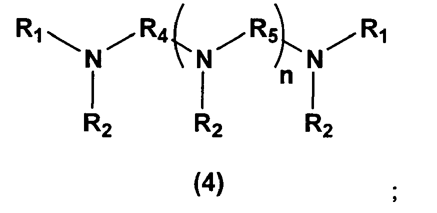 wherein R₁ is C₃ - C₁₂ branched alkyl, linear alkyl, alkenyl, cycloalkyl, or aralkyl; R₂ is selected from the group consisting of C₁ - C₄ alkyl, benzyl, C₃ - C₅ alkenyl, and (CH₂)_{P}-COO⁻ in which p is either 1 or 2; R₄ and R₅ are chosen independently from C₂ - C₆ alkylene; and n is an integer from 0 to 4; provided that n is ≥ 1 if R₂ is not (CH₂)ₚ-COO⁻.

27. The compound of claim 26, wherein R₄ and R₅ are both (CH₂)₂.

28. The compound of claim 26, wherein R₄ and R₅ are both (CH₂)₃.

29. The compound of claim 26, wherein R₄ and R₅ are both (CH₂)₆.

30. The compound of claim 26, wherein R₄ and R₅ are both (CH₂)₃CH(CH₃)CH₂.

31. A method of reducing corrosion of a metal surface, comprising contacting the metal surface with a corrosive medium comprising a corrosion inhibitor according to any of:
a) formula (1): 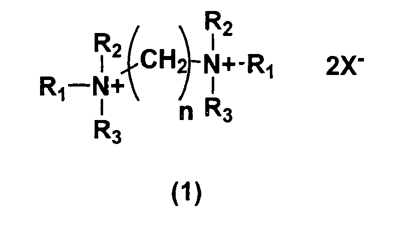 wherein R₁ is selected from the group consisting of C₄-C₁₈ branched alkyl, linear alkyl, alkenyl, cycloalkyl, aryl, alkaryl, and aralkyl; R₂ is selected from the group consisting of C₁-C₈ alkyl, alkenyl, cycloalkyl, aryl, alkaryl, aralkyl, and moieties of formula (A) 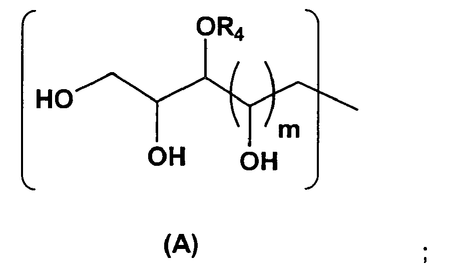 R₃ is C₁-C₈ alkyl, alkenyl, cycloalkyl, aryl, alkaryl, or (CH₂)_{P}-COO⁻ in which p is either 1 or 2; n is an integer from 2 to 14; m is an integer from 0 to 2; R₄ is H, α-D-glucopyranosyl, β-D-pyranosyl, or β-D-galactopyranosyl; and X⁻ is Cl⁻, Br⁻ , I⁻, OH⁻, CH₃COO-, 1/2 SO₄⁻², or 1/3 PO₄⁻³, provided that the number of X⁻ moieties in formula (1) is reduced by one for each R₃ that is (CH₂)ₚ-COO⁻; further provided that R₁ is not linear alkyl when R₃ is not (CH₂)ₚ-COO⁻;
b) formula (2): 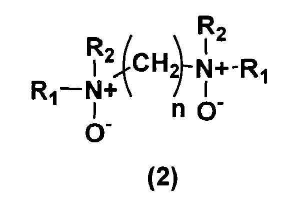 wherein R₁ is selected from the group consisting of C₄ - C₁₈ branched alkyl, linear alkyl, alkenyl, cycloalkyl, aryl, alkaryl, and aralkyl; R₂ is selected from the group consisting of C1 - C8 alkyl, alkenyl, cycloalkyl, aryl, alkaryl, aralkyl, and moieties of formula (A) 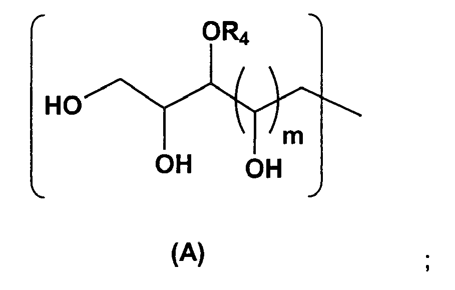 n is an integer from 2 to 14; m is an integer from 0 to 2; and R₄ is H, α-D-glucopyranosyl, β-D-pyranosyl, or β-D-galactopyranosyl;
c) formula (3): 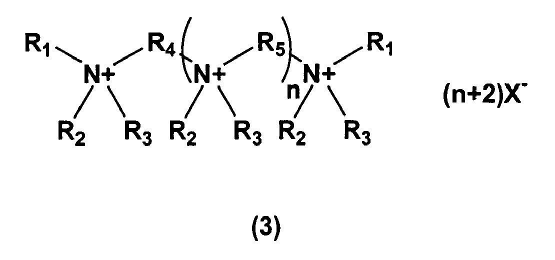 wherein R₁ is C₃ - C₁₂ branched alkyl, linear alkyl, alkenyl, cycloalkyl, or aryl-substituted branched or linear alkyl; R₂ and R₃ are each individually selected from the group consisting of C₁ - C₄ alkyl, benzyl, C₃ - C₅ alkenyl, and (CH₂)ₚ-COO⁻ in which p is either 1 or 2, provided that not both of R₂ and R₃ are (CH₂)ₚ-COO⁻; R₄ and R₅ are chosen independently from C₂ - C₆ alkylene; n is an integer from 0 to 4; and X⁻ is Cl⁻, Br⁻, I⁻, OH⁻, CH₃COO-, 1/2 SO₄⁻², or 1/3 PO₄⁻³, provided that the number of X⁻ moieties in formula (3) is reduced by one for each R₃ that is (CH₂)_{P}-COO-; further provided that, if n is 0 and R₄ is (CH₂)₂, then either R₁ is not linear alkyl or R₃ is (CH₂)ₚ-COO⁻, or both; and
d) formula (4): 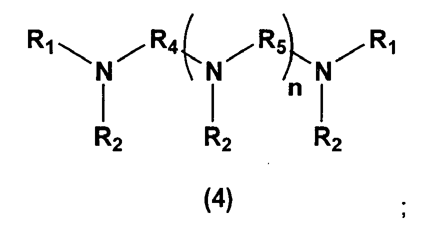 wherein R₁ is C₃ - C₁₂ branched alkyl, linear alkyl, alkenyl, cycloalkyl, or aralkyl; R₂ is selected from the group consisting of C₁ - C₄ alkyl, benzyl, C₃ - C₅ alkenyl, and (CH₂)ₚ-COO⁻ in which p is either 1 or 2; R₄ and R₅ are chosen independently from C₂ - C₆ alkylene; and n is an integer from 0 to 4; provided that n is ≥ 1 if R₂ is not (CH₂)_{P}-COO⁻.
